(19) Europäisches Patentamt
European Patent Office
Office européen des brevets

(11) Veröffentlichungsnummer: **0 203 033 B1**

(12) **EUROPÄISCHE PATENTSCHRIFT**

(45) Veröffentlichungstag der Patentschrift: **11.03.92**

(51) Int. Cl.[5]: **C10M 135/36**, C07D 277/70, C07D 417/12, //C10N30:06

(21) Anmeldenummer: **86810217.9**

(22) Anmeldetag: **20.05.86**

# (54) Aminomethylderivate von Benzothiazolinthion als Schmiermitteladditive.

(30) Priorität: **23.05.85 CH 2199/85**

(43) Veröffentlichungstag der Anmeldung:
**26.11.86 Patentblatt 86/48**

(45) Bekanntmachung des Hinweises auf die Patenterteilung:
**11.03.92 Patentblatt 92/11**

(84) Benannte Vertragsstaaten:
**BE CH DE FR GB IT LI NL**

(56) Entgegenhaltungen:

**CRC HANDBOOK OF LUBRIFICATION, Band 2, 1984, CRC Press, Inc. , Florida J.A. O BRIEN "Lubrificating Oil Additives" Seiten 301-315**

**J. ORG. CHEM., Band 36, Nr. 5, 1971, Washington, USA A.F. HALASA et al. "Study of the Michael and Mannich Reactions with Benzothiazole-2-thiol." Seiten 636-641**

(73) Patentinhaber: **CIBA-GEIGY AG**
**Klybeckstrasse 141**
**CH-4002 Basel(CH)**

(72) Erfinder: **Camenzin, Hugo, Dr.**
**Av. Général-Guisan 42**
**CH-1700 Fribourg(CH)**
Erfinder: **Phillips, Emyr, Dr.**
**12 Pinewood Court Broad Road**
**Sale Cheshire M33 2ES(GB)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft Schmiermittelzusammensetzungen, die öllösliche Derivate des Benzothiazolin-2-thion als Schmiermittelzusätze enthalten, und die Verbindungen selbst, soweit sie neu sind.

Mineralischen und synthetischen Schmiermitteln werden üblicherweise Zusatzstoffe zur Verbesserung der Gebrauchseigenschaften zugesetzt. Von besonderer Bedeutung sind Additive, welche die zu schmierende Vorrichtung vor Reibungsabnutzung schützen. An solche Additive wird die Anforderung gestellt, dass sie das Lasttragevermögen des Schmiermittels erhöhen, nicht korrodierend auf die zu schmierenden Metallteile wirken und eine gute Hitzebeständigkeit besitzen.

Hierfür werden vorzugsweise phosphor- und schwefelhaltige Verbindungen verwendet wie z.B. Salze von Dialkyldithiophosphaten (siehe dazu CRC Handbook of Lubrication, Vol. 2 (1984), 301-315, CRC Press Inc.). Im Hinblick auf die Verwendung von Katalysatoren im Abgassystem von Verbrennungsmotoren soll jedoch der Phosphorgehalt von Schmierölen möglichst tief gehalten werden, damit die Katalysatoren nicht desaktiviert werden [H.S. Gandhi et al., Applied Catalysis 3, (1982) 79-82].

Es wurde nun gefunden, dass bestimmte phosphorfreie und öllösliche Derivate des Benzothiazols in mineralischen und synthetischen Schmiermitteln ausgezeichnete Eigenschaften hinsichtlich Schutz vor Reibungsabnutzung, Lasttragevermögen, Schutz der Metallteile vor Korrosion und Aschefreiheit aufweisen. In Schmiermittelformulierungen, die reduzierte Mengen an Zinkdialkyldithiophosphaten enthalten, bewirken diese Derivate einen deutlich erhöhten Schutz gegen Oxidation. Es handelt sich dabei um N-Aminomethylderivate des Benzothiazolin-2-thion.

Die Erfindung betrifft daher die Schmiermittelzusammensetzungen enthaltend

a) eine oder mehrere Schmiermittel auf Basis von mineralischen oder synthetischen Oelen,

b) 0,05 bis 5 Gew.% mindestens einer Verbindung der Formel I

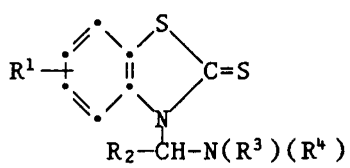

I

worin $R^1$ Wasserstoff, $C_1$-$C_{12}$-Alkyl, $C_2$-$C_4$-Alkoxy, $C_1$-$C_{24}$-Alkoxycarbonyl oder Nitro,

$R^2$ Wasserstoff, $C_1$-$C_{12}$-Alkyl, 2-Furyl, unsubstituiertes oder durch $C_1$-$C_4$-Alkyl, $C_1$-$C_4$-Alkoxy, $C_1$-$C_{24}$-Alkoxycarbonyl oder Nitro substituiertes Phenyl,

$R^3$ und $R^4$ unabhängig voneinander H, $C_1$-$C_{20}$-Alkyl, das durch ein oder mehrere O, S oder N unterbrochen sein oder Oxo- oder Thionogruppen enthalten kann, $C_3$-$C_{24}$-Alkenyl, $C_3$-$C_{12}$-Alkoxyalkyl, $C_5$-$C_8$-Cycloalkyl, unsubstituiertes oder durch $C_1$-$C_{12}$-Alkyl, $C_1$-$C_4$-Alkoxy, $C_1$-$C_{24}$-Alkoxycarbonyl oder Nitro substituiertes Phenyl, Naphthyl, $C_7$-$C_9$-Phenylalkyl, 2-Furylmethyl oder 2-(Tetrahydrofuryl)-methyl bedeuten, oder $R^3$ und $R^4$ zusammen mit dem N-Atom, an das sie gebunden sind, einen 5- oder 6-gliedrigen Ring bilden, der ausser dem N-Atom noch weitere Heteroatome (O, N, S) oder Oxo- oder Thionogruppen oder durch einen Benzorest anneliert sein kann, oder $R_4$ einen Rest der Formel

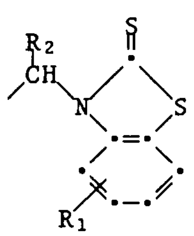

(II) oder

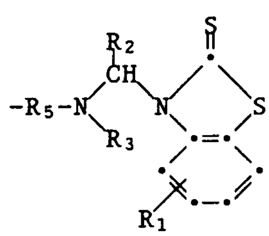

(III)

bedeutet, und

$R^5$ $C_2$-$C_{12}$-Alkylen, das durch O, S oder N unterbrochen sein oder Oxo- oder Thionogruppen enthalten kann, $C_6$-$C_{15}$-Cycloalkylen, $C_6$-$C_{15}$-Arylen, Carbonyl oder Thiocarbonyl bedeutet oder die Gruppe -N($R^3$)-$R^5$-N($R^3$)-einen Piperazin-1,4-diylrest, der durch eine oder mehrere Methylgruppen substituiert sein kann, bedeutet, als Reibung, Verschleiss, Korrosion und Oxidation vermindernde Zusätze für a); und

c) gegebenenfalls weitere Schmiermittelzusätze.

$R^1$, $R^2$, $R^3$ und $R^4$ als Alkyl können dabei unverzweigt oder verzweigt sein. Beispiele hierfür sind Methyl, Ethyl, Isopropyl, n-Butyl, sec.-Butyl, t-Butyl, Isoamyl, n-Hexyl, 2-Ethylbutyl, 1-Methylpentyl, 1,3-Dimethylbutyl, 2-Ethylhexyl, Isoheptyl, n-Octyl, 1-Methylheptyl, 1,1,3-Trimethylhexyl, n-Decyl, 1-Methylundecyl oder n-Dodecyl. $R^3$ und $R^4$ als Alkyl können darüber hinaus auch z.B. Tetradecyl, Hexadecyl, Octadecyl oder Eicosyl bedeuten.

$R^1$ und $R^2$ als Alkoxy können z.B. Methoxy, Ethoxy, Isopropoxy oder n-Butoxy sein.

$R^1$ als Alkoxycarbonyl enthält 1-24 Kohlenstoffatome im Alkylteil und kann z.B. Methoxycarbonyl, Ethoxycarbonyl oder 2-Ethylhexoxycarbonyl sein.

$R^2$, $R^3$ und $R^4$ können mit Alkoxycarbonyl substituiertes Phenyl sein, dann gilt für das Alkoxycarbonyl das für $R^1$ gesagte.

$R^2$, $R^3$ und $R^4$ können substituiertes Phenyl bedeuten, wie z.B. Tolyl, Xylyl, 4-t-Butylphenyl, 3-Methoxyphenyl, 4-Propoxyphenyl, 3-Butoxycarbonylphenyl, 3-Nitrophenyl oder 4-Methyl-3-nitrophenyl.

$R^3$ und $R^4$ als Alkoxyalkyl können z.B. 2-Methoxyethyl, 2-Isopropoxyethyl, 2-Butoxyethyl oder 2-Octyloxyethyl sein. $R^3$ und $R^4$ als Cycloalkyl können z.B. Cyclopentyl, Cyclohexyl, 4-Methylcyclohexyl oder Cyclooctyl sein. $R^3$ und $R^4$ als Phenylalkyl können z.B. Benzyl, 2-Phenylethyl, 1-Phenylethyl oder 2-Phenylpropyl sein. Wenn $R^3$ und $R^4$ zusammen mit dem N-Atom einen 5- oder 6-gliedrigen Ring bilden, so kann dies z.B. ein Pyrrolidin-, Piperidin, Morpholin-, Thiomorpholin-, Piperazin-, Indol-, Tetrahydrochinolin- oder Tetrahydroisochinolinring sein. $R^3$ und $R^4$ als Alkenyl können z.B. Allyl, Methallyl, 1-Pentenyl, Dodecenyl oder Octadecenyl sein.

$R^5$ als Alkylen kann unverzweigtes oder verzweigtes Alkylen sein oder auch durch O, S oder N unterbrochen sein. Beispiele hierfür sind Di-, Tri-, Tetra-, Hexa-, Octa-, Deca- oder Dodecamethylen; 2,2,4- oder 2,4,4-Trimethyl-hexamethylen, 3-Oxa-pentamethylen, 4-Thia-heptamethylen oder 4-(Methylaza)-heptamethylen.

$R^5$ als Cycloalkylen kann z.B. 1,4-Cyclohexylen, 1,4-Decahydronaphthylen, Cyclohexan-1,4-dimethylen oder Dicyclohexyl-methan-4,4-diyl sein. $R^5$ als Arylen kann z.B. 1,3-Phenylen, 1,4-Phenylen, 1,4-Naphthylen, 4,4'-Diphenylen, Diphenyl-methan-4,4'-diyl oder Diphenyloxid-4,4'-diyl sein.

Die Summe der in den Resten $R^1$, $R^2$, $R^3$, $R^4$ und $R^5$ enthaltenen C-Atome beträgt vorzugsweise mehr als 10, insbesondere mehr als 14. Diese Reste tragen zur Löslichkeit in Oel bei.

Bevorzugt sind Schmiermittelzusammensetzungen, welche 0,1 bis 3 Gew.% mindestens einer Verbindung der Formel I enthalten.

Bevorzugt sind Schmiermittelzusammensetzungen, welche mindestens eine Verbindung der Formel I enthalten, worin $R^2$ Wasserstoff ist, und insbesondere worin $R^1$ ebenfalls Wasserstoff ist.

Bevorzugt sind Schmiermittelzusammensetzungen, welche mindestens eine Verbindung der Formel I enthalten, worin $R^1$ und $R^2$ Wasserstoff sind, $R^3$ und $R^4$ unabhängig voneinander H, $C_1$-$C_{24}$-Alkyl, $C_3$-$C_{24}$-Alkenyl, Cyclohexyl, Phenyl, durch $C_1$-$C_{12}$-Alkyl oder $C_1$-$C_{24}$-Alkoxycarbonyl substituiertes Phenyl, $C_7$-$C_{10}$-Phenylalkyl, 2-Methoxyethyl oder 3-Methoxypropyl und $R^5$ $C_2$-$C_{12}$-Alkylen bedeuten.

Beispiele für erfindungsgemäss verwendbare Verbindungen der Formel I sind:

3-[Bis-(2-ethylhexyl)aminomethyl]-benzothiazolin-2-thion
3-(Dicyclohexylaminomethyl)-benzothiazolin-2-thion
3-(Diphenylaminomethyl)-benzothiazolin-2-thion
3-[Bis(4-dodecylphenyl)aminomethyl]-benzothiazolin-2-thion
3-[Dibenzylaminomethyl]-benzothiazolin-2-thion
3-[N-(1-Naphthylo)-phenylaminomethyl]-benzothiazolin-2-thion
3-[N-(2-Furylmethyl)-butylaminomethyl]-benzothiazolin-2-thion
3-[N-(2-Tetrahydrofurylmethyl)-hexylaminomethyl]-benzothiazolin-2-thion
3-[Bis(2-methoxyethyl)aminomethyl]-benzothiazolin-2-thion
3-(Piperidinomethyl)-benzothiazolin-2-thion
3-(Pyrrolidinomethyl)-benzothiazolin-2-thion
3-(Morpholinomethyl)-benzothiazolin-2-thion
3-[(2,3-Dihydroindolyl)methyl]-benzothiazolin-2-thion
3-[(1,2,3,4-Tetrahydrochinolyl)methyl]-benzothiazolin-2-thion
3-(Phenylaminomethyl)-benzothiazolin-2-thion
3-[(4-Dodecylphenyl)aminomethyl]-benzothiazolin-2-thion
3-[Bis()2-ethylhexyl)aminomethyl]-5-carbethoxy-benzothiazolin-2-thion.

N,N-Bis(2-thionobenzothiazolin-3-ylmethyl)-2-ethylhexylamin
N,N-Bis(2-thionobenzothiazolin-3-ylmethyl)-n-dodecylamin
N,N-Bis(2-thionobenzothiazolin-3-ylmethyl)-3-ethoxypropylamin.

N,N'-Bis(2-thionobenzothiazolin-3-ylmethyl)-N,N'-dimethyl-ethylendiamin,

N,N'-Bis(2-thionobenzothiazolin-3-ylmethyl)-piperazin
N,N'-Bis(2-thionobenzothiazolin-3-ylmethyl)-2,5-dimethylpiperazin
N,N'-Bis(2-thionobenzothiazolin-3-ylmethyl)-4,4'-diamino-dicyclohexylmethan
N,N'-Bis(2-thionobenzothiazolin-3-ylmethyl)-p-phenylendiamin.

Die Schmiermittel, für die man die genannten Additive verwenden kann, können Schmieröle oder Schmierfette auf Basis von Mineralölen oder von synthetischen Oelen oder deren Gemischen sein. Synthetische Oele können z.B. Esteröle, Olefinpolymerisate oder Ethylenoxidpolymerisate sein. Man kann die Additive auch in einer geringeren Menge des Oeles vorlösen und dieses Konzentrat dem Schmiermittel zumischen.

Ausser den Additiven der Formel I kann das Schmiermittel auch andere Additive enthalten, wie sie für Schmiermittel üblich sind. Dies können z.B. Antioxydantien, Metallpassivatoren, Korrosionsinhibitoren, Viskositätsindex-Verbesserer, Stockpunkterniedriger, Dispergiermittel, Detergentien oder andere Verschleissschutz-Additve sein. Im Falle von Schmierfetten werden den Oelen Verdickungsmittel zugesetzt.

Beispiele für Antioxidantien sind:

a) Alkylierte und nicht-alkylierte aromatische Amine und Mischungen davon z.B. Dioctyldiphenylamin, Mono-t-octylphenyl-$\alpha$- und -$\beta$-naphthylamine, Phenothiazin, Dioctylphenothiazin, Phenyl-$\alpha$-naphthylamin, N,N'-Di-sec.-butyl-p-phenylendiamin.

b) Sterisch gehinderte Phenole, z.B. 2,6-Di-tert.-butyl-p-cresol, 4,4'-Bis-(2,6-diisopropylphenol), 2,4,6-Triisopropylphenol, 2,2'-Thio-bis-(4-methyl-6-tert.-butylphenol), 4,4'-Methylen-bis(2,6-di-tert.-butyl-phenol).

c) Ester von Thiodipropionsäure oder Thiodiessigsäure, z.B. Dilaurylthiodipropionat oder Dioctylthiodiacetat.

d) Salze von Carbamin- und Dithiophosphorsäuren, z.B. Antimon-diamyldithiocarbamat, Zink-diamyldithiophosphat.

Beispiele für Metallpassivatoren sind:

a) für Kupfer, z.B. Benzotriazol, Tetrahydrobenzotriazol, 2-Mercaptobenzotriazol, 2,5-Dimercaptothiadiazol, Salicylidenpropylendiamin, Salze von Salicylaminoguanidin.

b) für Blei, z.B. Sebacinsäurederivate, Chinizarin, Propylgallat.

Beispiele für Rost-Inhibitoren sind:

a) Organische Säuren, ihre Ester, Metallsalze und Anhydride, z.B. H-Oleoyl-sarcosin, Sorbitan-monooleat, Blei-naphthenat, Dodecenylbernsteinsäure-anhydrid.

b) Stickstoffhaltige Verbindungen, z.B. primäre, sekundäre oder tertiäre aliphatische oder cylcoaliphatische Amine und Amin-Salze von organischen und anorganischen Säuren, z.B. öllösliche Alkylammoniumcarboxylate. Heterocyclische Verbindungen, z.B. substituierte Imidazoline und Oxazoline.

c) Schwefelhaltige Verbindungen, z.B. Barium-dinonylnaphthalinsulfonate, Calciumpetroleumsulfonate.

Beispiele für Viskositätsindex-Verbesserer sind z.B.:
Polymethacrylate, Vinylpyrrolidon/Methacrylat-Copolymere, Polybutene, Olefin-Copolymere, Styrol/Acrylat-Copolymere.

Beispiele für Stockpunkterniedriger sind z.B.:
Alkylierte Naphthaline, alkylierte Phenole, Polymethacrylate.

Beispiele für Detergentien und Dispergiermittel sind:
Polyalkenylbernsteinsäureamide, öllösliche Metallseifen wie Ca-, Ba-, Mg-und Al-Carboxylate, -Phenolate oder -Sulfonate.

Beispiele für andere Verschleisschutz-Additive sind z.B.:
Schwefel und/oder Phosphor und/oder Halogen enthaltende Verbindungen, wie geschwefelte vegetabilische Oele, Zinkdialkyldithiophosphate, Tritolyl-phosphat, chlorierte Paraffine, Alkyl- und Aryldisulfide.

Die Verbindungen der Formel I sind z.T. bekannt und z.T. neue Verbindungen. Bekannt sind Verbindungen der Formel I, worin $R^1$ und $R^2$ Wasserstoff sind und $R^3$ und $R^4$ zusammen nicht mehr als 14 C-Atome enthalten und $R_4$ nicht ein Rest der Formeln II oder III ist. Solche Verbindungen wurden durch eine Mannich-Reaktion von 2-Mercaptobenzothiazol mit Formaldehyd und einem primären oder sekundären Amin hergestellt.

Halasa und Smith/J. Org. Chem 36 (1971), 636-41, haben bewiesen, dass unter den für Mannich-Reaktionen üblichen Bedingungen die Aminomethylierung von 2-Mercaptobenzothiazol am N-Atom der tautomeren Form des Benzothiazolin-2-thion erfolgt.

Gegenstand der Erfindung sind auch Verbindungen der Formel I

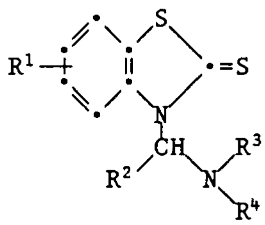

(I),

worin $R^1$, $R^2$, $R^3$ und $R^4$ die oben angegebenen Bedeutungen haben, mit der Massgabe, dass -$NR^3R^4$ nicht Piperidino, Pyrrolidino, N,N-Diethylamino, Benzylamino, Cyclohexylamino oder Morpholino bedeuten, wenn $R^1$ und $R^2$ Wasserstoff sind.

Bevorzugt sind Verbindungen der Formel I mit obiger Massgabe, worin $R^2$ Wasserstoff ist, ferner solche worin $R^1$ und $R^2$ Wasserstoff sind und $R^3$ und $R^4$ zusammen mehr als 10 C-Atome enthalten, insbesondere solche, worin $R^3$ und $R^4$ unabhängig voneinander $C_6$-$C_{12}$-Alkyl oder $C_5$-$C_6$-Cycloalkyl bedeuten, vor allem solche, worin $R^3$ und $R^4$ 2-Ethylhexyl sind.

Ganz besonders bevorzugt sind auch Verbindungen der Formel I, worin $R^1$ und $R^2$ Wasserstoff sind, $R^4$ eine Gruppe der Formel III darstellt und $R^5$ nicht Arylen ist, vor allem auch solche, worin $R^3$ Wasserstoff, $C_1$-$C_{24}$-Alkyl oder $C_3$-$C_{24}$-Alkenyl ist und $R^5$ $C_2$-$C_{12}$-Alkylen bedeutet.

Die Verbindungen der Formel I, worin $R^4$ nicht Reste der Formeln II oder III bedeutet, werden durch Reaktion eines 2-Mercaptobenzothiazols IV mit einem Monoamin $R^3$-NH-$R^4$ und einem Aldehyd $R^2$-CHO hergestellt.

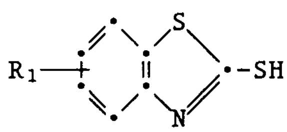 + $R^2$-CHO + $R^3$-NH-$R^4$ ⟶ I

(IV)

Das Amin kann ein primäres oder sekundäres Monoamin sein. Man arbeitet dabei in annährend equimolarem Verhältnis oder mit einem geringen Ueberschuss an Aldehyd. Als Reaktionsmedium werden unpolare Lösungsmittel bevorzugt, insbesondere Toluol, Cyclohexan oder Ligroin. Man kann aber auch polare Lösungsmittel, insbesondere Alkohole wie Ethanol oder Methanol, verwenden. Man kann auch - wie es Halasa und Smith beschrieben haben - zuerst das entsprechende Aminsalz von IV bilden, das in Wasser löslich ist, und anschliessend das Salz mit dem Aldehyd im wässrigen Medium umsetzen.

Die Verbindungen der Formel I, worin $R_4$ einen Rest der Formel II bedeutet, werden durch Reaktion von IV mit $R^2$-CHO und einem primären Monoamin $R^3$-$NH_2$ im molaren Verhältnis 2:2:1 hergestellt:

2 IV + 2 $R^2$-CHO + $R^3$-$NH_2$ ⟶ 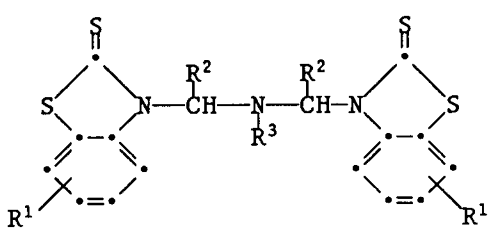

Die Verbindungen der Formel I, worin $R_4$ einen Rest der Formel III bedeutet, werden durch entsprechende Mannich-Reaktion unter Verwendung eines Diamines $R^3$-NH-$R^5$-NH-$R^3$ hergestellt:

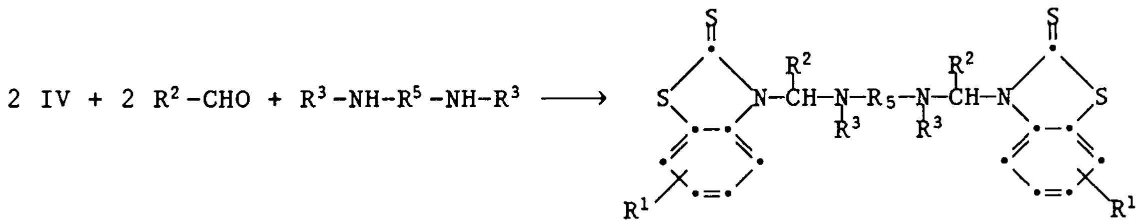

Das Diamin kann ein primäres oder sekundäres Diamin sein, bevorzugt verwendet man ein primäres Diamin ($R^3$ = H).

Die Ausführung der Erfindung wird im folgenden an Hand einzelner Beispiele beschrieben. Die Temperaturen sind in ° C angegeben.

Beispiel 1: Mannich-Reaktion in Methanol/Wasser

Zu einer Suspension von 250,8 g (1,5 Mol) 2-Mercaptobenzothiazol in 1500 ml Methanol werden 112,8 ml 37%iger wässriger Formaldehyd (1,5 Mol) zugegeben und unter Rühren 362,4 g (1,5 Mol) Bis(2-ethylhexyl)amin zugetropft. Die Mischung wird 15 h bei 50° gerührt und dann im Vakuum eingedampft. Es hinterbleiben 618 g rohes 3-[Bis(2-ethylhexyl)aminomethyl]-benzothiazolin-2-thion als rotbraunes Oel.

| | | | | |
|---|---|---|---|---|
| Analyse berechnet | C 68,52 % | H 9,58 % | N 6,66 % | S 15,24 % |
| gefunden | 68,36 % | 9,57 % | 6,69 % | 15,29 % |

Beispiel 2: Mannich-Reaktion in Ethanol/Wasser

In eine Suspension von 33,45 g (0,2 Mol) 2-Mercaptobenzothiazol in 120 ml Ethanol werden unter Rühren 18,63 g (0,2 Mol) Anilin zugegeben wobei die Temperatur auf 30° steigt. Dann werden 16,4 g 37%ige wässrige Formaldehydlösung (0,2 Mol) zugesetzt und weitere 30 Minuten gerührt. Der gebildete Niederschlag wird abfiltriert und mit kaltem Ethanol und Hexan gewaschen. Man erhält so 47 g 3-(Phenylaminomethyl)-benzothiazolin-2-thion, das bei 102-103° schmilzt.

| | | | | |
|---|---|---|---|---|
| Analyse berechnet | C 61,74 % | H 4,44 % | N 10,29 % | S 23,54 % |
| gefunden | 61,53 % | 4,43 % | 10,10 % | 23,79 % |

In analoger Weise wird bei Verwendung von technischem Dodecylanilin (Isomerengemsich) das 3-(Dodecylphenylaminomethyl)-benzothiazolin-2-thion als braunes Oel erhalten.

Beispiel 3: Mannich-Reaktion in Toluol/Wasser

Zu einer Suspension von 16,7 g (0,1 Mol) 2-Mercaptobenzothiazol in 40 ml Toluol werden 10,12 g (13,7 ml; 0,01 Mol) Dipropylamin und 8,3 g (7,6 ml; 0,1 Mol) 36%iger wässriger Formaldehyd zugetropft. Die Mischung wird während 5 Stunden bei 50° kräftig gerührt. Dann wird die kleine Wasserphase abgetrennt, und die Toluolphase wird in Vakuum eingedampft. Es verbleiben 26,4 g eines gelb-orangen Kristallisates von 3-(Dipropylaminomethyl)-benzothiazolin-2-thion, das bei 60-62° schmilzt.

| | | | | |
|---|---|---|---|---|
| Analyse berechnet | C 59,96 % | H 7,19 % | N 9,99 % | S 22,86 % |
| gefunden | 59,82 % | 7,20 % | 9,89 % | 22,86 % |

In analoger Weise werden weitere Beispiele hergestellt, die aus den nachfolgenden Tabellen 1-3 ersichtlich sind. Dabei bedeuten:

Methode A: analog Beispiel 1
Methode B: analog Beispiel 2

Methode C: analog Beispiel 3

Ferner bedeutet in der Spalte "Analyse" die jeweilige erste Zeile die "berechneten" Werte und die zweite die "gefundenen" Werte in Prozenten.

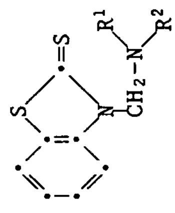

TABELLE 1

| Beispiel | 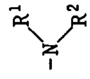 | Herst. Methode | Ausbeute (g) | Aspekt | Smp. (°C) | Analyse (%) C | H | N | S |
|---|---|---|---|---|---|---|---|---|---|
| 1 | $-N[CH_2-CH(CH_2CH_3)-(CH_2)_3-CH_3]_2$ | A/C | 98/97 | dunkelgelbes, zähflüssiges Oel | - | 68,52<br>68,36 | 9,58<br>9,57 | 6,66<br>6,69 | 15,24<br>15,29 |
| 2 | -NH- 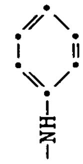 | B | 89 | beiges Kristallisat | 102-103 | 61,74<br>61,53 | 4,44<br>4,43 | 10,29<br>10,10 | 23,54<br>23,79 |
| 3 | $-N-(CH_2CH_2CH_3)_2$ | C | 94 | gelb-organges Kristallisat | 60-62 | 59,96<br>59,82 | 7,19<br>7,20 | 9,99<br>9,89 | 22,86<br>22,86 |
| 4 | Gemisch:<br>$-NHC_{14}H_{27}$<br>$-NHC_{12}H_{25}$ | C | 95 | braunes Oel | - | 66,62<br>66,51 | 9,05<br>9,25 | 7,40<br>7,62 | 16,93<br>16,96 |
| 5 | -NH- 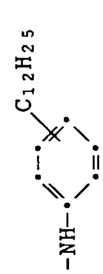<br>Isomerengemisch | B | 92 | dunkelbraunes, zähflüssiges Oel | - | 70,86<br>71,22 | 8,23<br>8,33 | 6,36<br>6,14 | 14,55<br>14,19 |
| 6 | $-N-(CH_2CH_2CH_2CH_3)_2$ | C | 92 | braunes Oel | - | 62,29<br>62,32 | 7,84<br>7,83 | 9,08<br>8,91 | 20,78<br>20,87 |

TABELLE 1 (Fortsetzung)

| Beispiel | 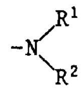 | Herst. Methode | Ausbeute (g) | Aspekt | Smp. (°C) | Analyse (%) C | H | N | S |
|---|---|---|---|---|---|---|---|---|---|
| 7 | 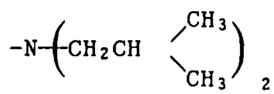 | C | 91 | beiges Kristallisat | 102-106 | 62,29<br>62,89 | 7,84<br>7,66 | 9,08<br>9,07 | 20,78<br>21,05 |
| 8 | $-N-(C_{13}H_{27})_2$ | C | 97 | braun-gelbes Oel | - | 72,80<br>72,60 | 10,78<br>10,73 | 4,99<br>4,88 | 11,43<br>11,41 |
| 9 | $-N-(C_{18}H_{37})_2$ | C | 99 | gelbes Kristallisat | 65 | 75,36<br>75,56 | 11,50<br>11,54 | 3,99<br>3,98 | 9,14<br>9,16 |
| 10 | 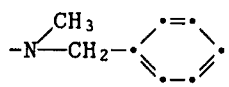 | B | 95 | braunes, zähflüssiges Oel | - | 63,97<br>63,93 | 5,37<br>5,42 | 9,32<br>9,13 | 21,34<br>21.10 |
| 11 | 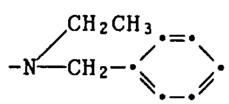 | B | 92 | gelbes Kristallisat | 85-87 | 64,93<br>64,52 | 5,77<br>5,75 | 8,76<br>8,76 | 29,39<br>21,08 |
| 12 | 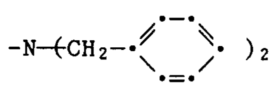 | B | 90 | gelbes Kristallisat | 96-98 | 70,18<br>70,05 | 5,35<br>5,43 | 7,44<br>7,37 | 17,03<br>17,22 |

TABELLE 1 (Fortsetzung)

| Bei-spiel | 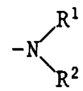 | Herst. Methode | Ausbeute (g) | Aspekt | Smp. (°C) | Analyse (%) C | H | N | S |
|---|---|---|---|---|---|---|---|---|---|
| 13 | 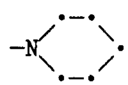 | B | 97 | gelbes Kristallisat | 152-156 | 59,05<br>59,04 | 6,10<br>6,10 | 10,60<br>10,27 | 24,25<br>24,26 |
| 14 | 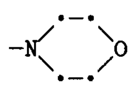 | B | 79 | gelbes Kristallisat | 134-137 | 54,11<br>54,12 | 5,30<br>5,25 | 10,52<br>10,01 | 24,07<br>24,77 |
| 15 | $-N(CH_2CH_2-O-CH_3)_2$ | B | 96 | braun-gelbes Oel | - | 53,82<br>53,93 | 6,45<br>6,51 | 8,97<br>8,75 | 20,52<br>19,91 |

TABELLE 2

| Beispiel | Verbindung | Herst. Methode | Ausbeute (g) | Aspekt | Analyse (%) C | H | N | S |
|---|---|---|---|---|---|---|---|---|
| 16 | 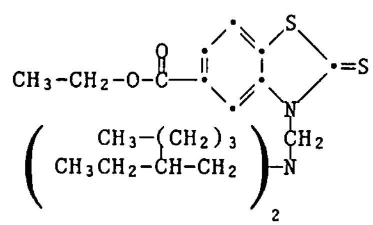 | C | 95 | braunes Oel | 65,81<br>65,95 | 9,00<br>9,22 | 5,68<br>5,59 | 13,01<br>12,90 |
| 17 | 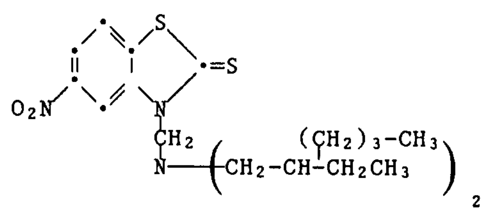 | C | 95 | orange-rotes Oel | 61,90<br>62,12 | 8,44<br>8,56 | 9,02<br>8,97 | 13,77<br>13,55 |

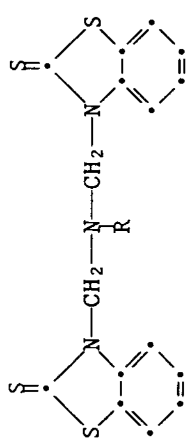

TABELLE 3

| Beispiel | - N - R | Herst. Methode | Ausbeute (g) | Aspekt | Smp. (°C) | Analyse (%) C | H | N | S |
|---|---|---|---|---|---|---|---|---|---|
| 18 | $-N-CH_2-\underset{CH_3}{\underset{\vert}{\underset{CH_2}{\underset{\vert}{CH}}}}-(CH_2)_3-CH_3$ | C | 97 | braunes, zähflüssiges Oel | - | 59,10<br>59,09 | 5,99<br>6,08 | 8,62<br>8,27 | 26,29<br>25,81 |
| 19 | $N-C_{18}H_{37}$ | C | 88 | gelbes Kristallisat | 90-92 | 65,02<br>65,19 | 7,86<br>7,94 | 6,69<br>6,52 | 20,42<br>20,31 |
| 20 | $N-C_{18}H_{35}$ | C | 99 | blassgelbes Wachs | - | -<br>- | -<br>- | -<br>6,68 | -<br>20,35 |
| 21 | $N-CH_2-$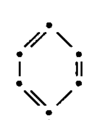 | C | 93 | gelbes Kristallisat | 158-163 | 59,32<br>61,14 | 4,11<br>4,54 | 9,02<br>8,34 | 27,54<br>25,80 |

Beispiel 22: Test auf Verschleissschutz

Zur Prüfung auf die Eignung auf Verschleissschutz wird die ASTM-Standard-MethodeD 2783-81 unter Verwendung des Shell-Vierkugelapparates herangezogen. Als Basisöl wird Catenex® P 941 der Fa. Shell

verwendet. Ermittelt werden a) die Schweisslast WL (Weld Load) als die Last (in kg) bei der die 4 Kugeln innerhalb von 10 Sekunden zusammenschweissen, und b) der mittlere Verschleiss-Durchmesser WSD (Wear Scar Diameter) bei einer Last von 40 kg während 1 Stunde (in mm).

Bei allen Testproben wird 1 Gew.% des Additivs eingesetzt.

Die Ergebnisse sind in Tabelle 4 zusammengefasst.

TABELLE 4

| Additiv aus Beispiel Nr. | WL (kg) | WSD (mm) |
|---|---|---|
| 1 | 180 | 0,55 |
| 3 | 200 | 0,60 |
| 5 | 180 | 0,55 |
| 6 | 180 | 0,55 |
| 7 | 180 | 0,60 |
| 8 | 200 | 0,55 |
| 9 | 180 | 0,60 |
| 10 | 180 | 0,60 |
| 12 | 180 | 0,60 |
| 15 | 180 | 0,65 |
| 16 | 180 | 0,55 |
| 17 | 200 | 0,50 |
| 19 | 180 | 0,60 |
| 20 | 200 | 0,60 |
| - | 160 | 0,90 |

Beispiel 23: Test auf Kupferpassivierung

Ein blank poliertes Kupferblech von 60 x 10 1 mm wird in Turbineöl getaucht, das 50 ppm gelösten Schwefel sowie 0,05 % 3-[Bis(2-ethylhexyl)aminomethyl]-benzothiazolin-2-thion enthält. Eine Vergleichsprobe enthält kein Thiazolinderivat. Die Proben werden 2 Stunden auf 100 ° C erwärmt. Anschliessend wird das Kupferblech mit Petrolether gewaschen, getrocknet und die Farbe des Bleches nach ASTM D 130 beurteilt durch Vergleich mit einer Standard-Farbtabelle. Die Beurteilung geschieht in 4 Stufen:

1 - kein Beschlag
2 - mässiger Beschlag
3 - starker Beschlag
4 - Korrosion

Ergebnis: Farbe der Probe 1 B
Vergleichsprobe 3 B

Beispiel 24: Test auf Stabilisierung gegen Oxidation
(TFOUT-Test: Thin-Film Oxygen Uptake Test)

Dieser Test ist eine modifizierte Version des "Rotary Bomb Oxidationstests für Mineralöle" (ASTM D 2272). Er wird genau beschrieben in "C.S. Ku und S.M. Hsu, A Thin-Film Oxygen Uptake Test for the Evaluation of Automotive Crankcase Lubricants, Lubrication Engineering, Vol 40 (2), 75-83 (1984)". Als Testöl dient ein Motorenöl auf Mineralölbasis, welches die Hälfte der üblichen Menge an Zinkdithiophosphat (0,75 %: Zinkgehalt 0,06 %, bezogen auf das Motorenöl) enthält; diese Aenderung wurde vorgenommen, damit ein potentieller Effekt des zu prüfenden Stabilisators gezeigt werden kann.

Als Basis werden zwei handelsübliche 15 W40-Motorenöle mit eingestelltem Zinkdithiophosphat-Gehalt (TDTP) verwendet:

Oel A: 0,063 % Phosphor in Form von primärem und sekundärem ZDTP (im Verhältnis 1:1).
Oel B: 0,067 % Phosphor in Form von primärem und sekundärem ZDTP (im Verhältnis 2:1).

Das in Beispiel 1 hergestellte Additiv wird im beschriebenen Motorenöl in Gegenwart von 2 % Wasser, einer flüssigen oxidierten, nitrierten Fraktion eines Motorenbenzins als Katalysator (4 % Einsatzkonzentration) eines flüssigen Metallnaphthenates als weiterem Katalysator (4 % Einsatzkonzentration; Wasser und

die beiden flüssigen Katalysatorsubstanzen sind unter der No. Standard Reference Material 1817 vom National Bureau of Standards (NBS) mit Analysen-Zertifikat geliefert worden) getestet. Der Versuch ist bei einem deutlichen Knick des Druck/Zeit-Diagramms beendet. Die in der untenstehenden Tabelle angegebnen Resultate bedeuten die Zeit (in Minuten) bis zum Knick des Druck/Zeit-Diagramms.

Lange Zeiten entsprechen guter Stabilisatorwirksamkeit. Konzentration des Stabilisators: 0,5 Gew.-%, bezogen auf das Oel.

| Formulierung | Induktionsperiode (Zeit bis zum deutlichen Druckabfall in Minuten) |
|---|---|
| Oel A | 95 |
| Oel A + 0,5 % Additiv | 170 |
| Oel B | 105 |
| Oel B + 0,5 % Additiv | 170 |

## Patentansprüche

1. Schmiermittelzusammensetzung enthaltend
   a) eine oder mehrere Schmiermittel auf Basis von mineralischen oder synthetischen Oelen,
   b) 0.05 bis 5 Gew.% mindestens einer Verbindung der Formel I,

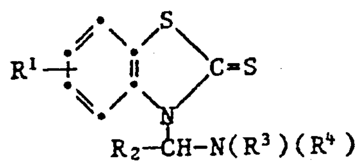

I

worin $R^1$ Wasserstoff, $C_1$-$C_{12}$-Alkyl, $C_2$-$C_4$-Alkoxy, $C_1$-$C_{24}$-Alkoxycarbonyl oder Nitro,
$R^2$ Wasserstoff, $C_1$-$C_{12}$-Alkyl, 2-Furyl, unsubstituiertes oder durch $C_1$-$C_4$-Alkyl, $C_1$-$C_4$-Alkoxy, $C_1$-$C_{24}$-Alkoxycarbonyl oder Nitro substituiertes Phenyl,
$R^3$ und $R^4$ unabhängig voneinander H, $C_1$-$C_{20}$-Alkyl, das durch ein oder mehrere O, S oder N unterbrochen Sein oder Oxo- oder Thionogruppen enthalten kann, $C_3$-$C_{24}$-Alkenyl, $C_3$-$C_{12}$-Alkoxyalkyl, $C_5$-$C_8$-Cycloalkyl, unsubstituiertes oder durch $C_1$-$C_{12}$-Alkyl, $C_1$-$C_4$-Alkoxy, $C_1$-$C_{24}$-Alkoxycarbonyl oder Nitro substituiertes Phenyl, Naphthyl, $C_7$-$C_9$-Phenylalkyl, 2-Furylmethyl oder 2-(Tetrahydrofuryl)-methyl bedeuten, oder $R^3$ und $R^4$ zusammen mit dem N-Atom, an das sie gebunden sind, einen 5- oder 6-gliedrigen Ring bilden, der ausser dem N-Atom noch weitere Heteroatome (O, N, S) oder Oxo- oder Thionogruppen oder durch einen Benzorest anneliert sein kann,
oder $R_4$ einen Rest der Formel

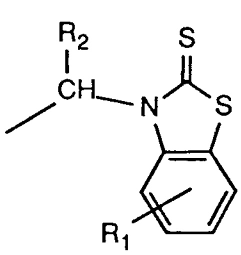

(II) oder

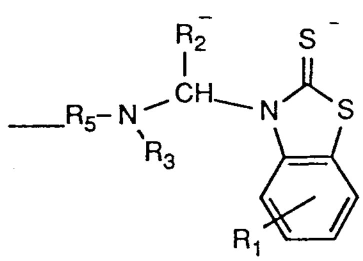

(III)

bedeutet,
$R^5$ $C_2$-$C_{12}$-Alkylen, das durch O, S oder N unterbrochen sein oder Oxo- oder Thionogruppen enthalten kann, $C_6$-$C_{15}$-Cycloalkylen, $C_6$-$C_{15}$-Arylen, Carbonyl oder Thiocarbonyl bedeutet oder die Gruppe -N($R^3$)-$R^5$-N($R^3$)- oder einen Piperazin-1,4-diylrest, der durch eine oder mehrere Methylgruppen substituiert sein kann, bedeutet, als Reibung, Verschleiss, Korrosion und Oxidation vermindernde Zusätze für a); und

c) gegebenenfalls weitere Schmiermittelzusätze.

**2.** Schmiermittelzusammensetzung gemäss Anspruch 1, enthaltend 0,1 bis 3 Gew.% mindestens einer Verbindung der Formel I.

**3.** Schmiermittelzusammensetzung gemäss Anspruch 1, enthaltend eine Verbindung der Formel I, worin die Summe der in $R^1$, $R^2$, $R^3$, $R^4$ und $R^5$ enthaltenen C-Atome grösser als 10 ist.

**4.** Schmiermittelzusammensetzung gemäss Anspruch 1, enthaltend eine Verbindung der Formeln I, worin $R^2$ Wasserstoff ist.

**5.** Schmiermittelzusammensetzung gemäss Anspruch 4, worin $R^1$ Wasserstoff ist.

**6.** Schmiermittelzusammensetzung gemäss Anspruch 3, worin $R^3$ und $R^4$ unabhängig voneinander H, $C_1$-$C_{24}$-Alkyl, $C_3$-$C_{24}$-Alkenyl, Cyclohexyl, Phenyl, durch $C_1$-$C_{12}$-Alkyl oder $C_1$-$C_{24}$-Alkoxycarbonyl substituiertes Phenyl, $C_7$-$C_{10}$-Phenylalkyl, 2-Methoxyethyl oder 3-Methoxypropyl, und $R^5$ $C_2$-$C_{12}$-Alkylen bedeuten.

**7.** Schmiermittelzusammensetzung gemäss Anspruch 1, enthaltend eine Verbindung der Formel

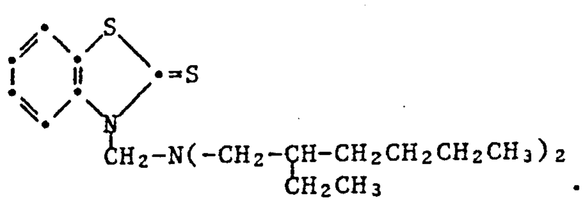

.

**8.** Schmiermittelzusammensetzung gemäss Anspruch 1, enthaltend eine Verbindung der Formel

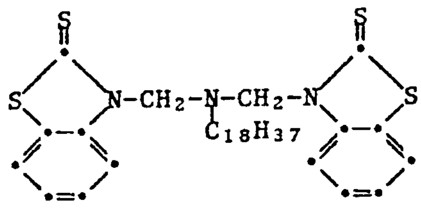

.

**9.** Schmiermittelzusammensetzung gemäss Anspruch 1, enthaltend eine Verbindung der Formel

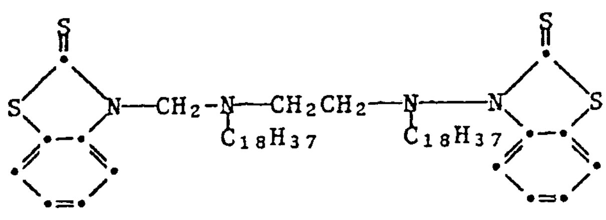

.

**10.** Verbindung der Formel I

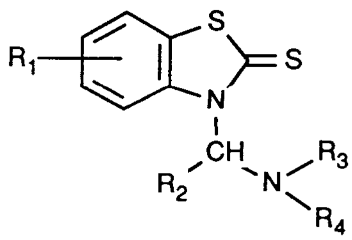

(I) ,

worin $R_1$, $R_2$, $R_3$ und $R_4$ die in Anspruch 1 angegebenen Bedeutungen haben , mit der Maßgabe, daß $-NR_3R_4$ nicht Piperidino, Pyrrolidino, N,N-Diethylamino, Benzylamino, Cyclohexylamino oder Morpholino bedeuten, wenn $R_1$ und $R_2$ Wasserstoff sind.

**11.** Verbindung der Formel I gemaß Anspruch 10, worin $R_2$ Wasserstoff ist.

**12.** Verbindung der Formel I gemaß Anspruch 10, worin $R_1$ und $R_2$ Wasserstoff sind und $R_3$ und $R_4$ zusammen mehr als 10 C-Atome enthalten.

**13.** Verbindung der Formel I gemaß Anspruch 12, worin $R_3$ und $R_4$ unabhängig voneinander $C_6$-$C_{12}$-Alkyl oder $C_5$-$C_6$-Cycloalkyl bedeuten.

**14.** Verbindung der Formel I gemaß Anspruch 13, worin $R_3$ und $R_4$ 2-Ethylhexyl sind.

**15.** Verbindung der Formel I gemäß Anspruch 10, worin $R_1$ und $R_2$ Wasserstoff sind, $R_4$ eine Gruppe der Formel III darstellt und $R_5$ nicht Arylen ist.

**16.** Verbindung der Formel I gemäß Anspruch 15, worin $R_3$ Wasserstoff, $C_1$-$C_{24}$-Alkyl oder $C_3$-$C_{24}$-Alkenyl ist und $R_5$ $C_2$-$C_{12}$-Alkylen bedeutet.

## Claims

**1.** A lubricant composition comprising
a) one or more lubricants based on mineral oils or synthetic oils,
b) 0.05 to 5 % by weight of at least one compound of formula I

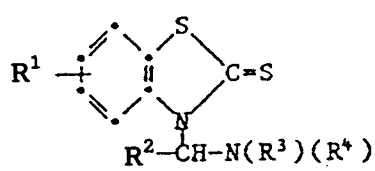

I

in which $R^1$ is hydrogen, $C_1$-$C_{12}$alkyl, $C_2$-$C_4$alkoxy, $C_1$-$C_{24}$alkoxycarbonyl or nitro,
$R^2$ is hydrogen, $C_1$-$C_{12}$alkyl, 2-furyl, phenyl which is unsubstituted or which is substituted by $C_1$-$C_4$alkyl, $C_1$-$C_4$alkoxy, $C_1$-$C_{24}$alkoxycarbonyl or nitro,
$R^3$ and $R^4$ are each independently of the other H, $C_1$-$C_{20}$alkyl which may be interrupted by one or more of O, S or N or may contain oxo or thiono groups, or are $C_3$-$C_{24}$alkenyl, $C_3$-$C_{12}$alkoxyalkyl, $C_5$-$C_8$cycloalkyl, phenyl which is unsubstituted or which is substituted by $C_1$-$C_{12}$alkyl, $C_1$-$C_4$alkoxy, $C_1$-$C_{24}$alkoxycarbonyl or nitro, or naphthyl, $C_7$-$C_9$phenylalkyl, 2-furylmethyl or 2-(tetrahydrofuryl)methyl; or $R^3$ and $R^4$, together with the N atom to which they are attached, form a 5- or 6-membered ring which, in addition to the N atom, may also contain further heteroatoms (O, N, S) or oxo or thiono groups, or may be fused on to a benzene nucleus, or $R^4$ is a radical of formula II and III

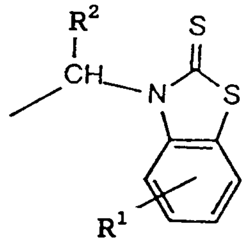 (II) or 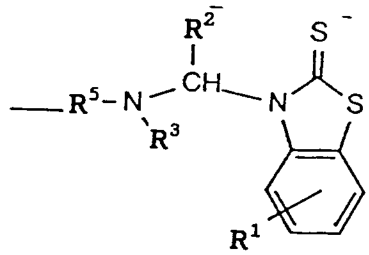 (III)

$R^5$ is $C_2$-$C_{12}$alkylene which may be interrupted by O, S or N or may contain oxo or thiono groups, or is $C_6$-$C_{15}$cycloalkylene, $C_6$-$C_{15}$arylene, carbonyl or thiocarbonyl or is the group -N($R^3$)-$R^5$-N($R^3$)- or a piperazine-1,4-diyl radical which may be substituted by one or more methyl groups, as additives for a) which reduce friction, wear, corrosion and oxidation;
and
c) optionally further lubricant additives.

**2.** A lubricant composition according to claim 1, comprising 0.1 to 3 % by weight of at least one compound of formula I.

**3.** A lubricant composition according to claim 1, which contains a compound of formula I in which the sum of the C atoms contained in $R^1$, $R^2$, $R^3$, $R^4$ and $R^5$ is greater than 10.

**4.** A lubricant composition according to claim 1, which contains a compound of formula I in which $R^2$ is hydrogen.

**5.** A lubricant composition according to claim 4, in which $R^1$ is hydrogen.

**6.** A lubricant composition according to claim 3, in which $R^3$ and $R^4$ are each independently of the other H, $C_1$-$C_{24}$alkyl, $C_3$-$C_{24}$alkenyl, cyclohexyl, phenyl, phenyl which is substituted by $C_1$-$C_{12}$alkyl or $C_1$-$C_{24}$alkoxycarbonyl, or are $C_7$-$C_{10}$phenylalkyl, 2-methoxyethyl or 3-methoxypropyl, and $R^5$ is $C_2$-$C_{12}$alkylene.

**7.** A lubricant composition according to claim 1, which contains a compound of formula

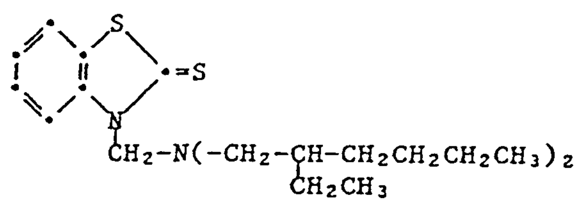 .

**8.** A lubricant composition according to claim 1, which contains a compound of formula

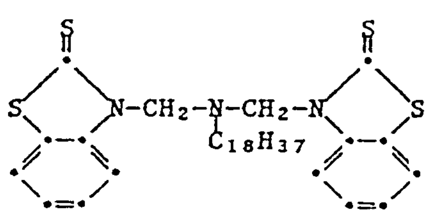 .

**9.** A lubricant composition according to claim 1, which contains a compound of formula

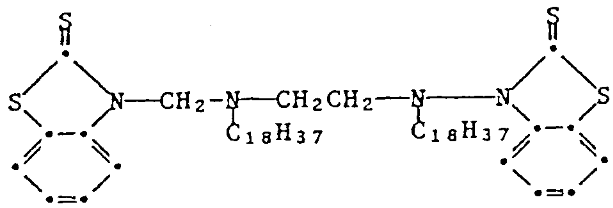

.

**10.** A compound of formula I

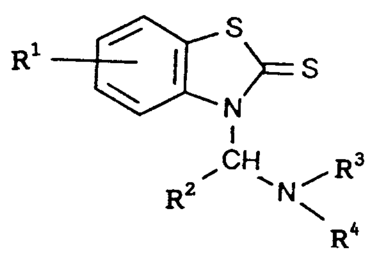

(I) ,

in which $R^1$, $R^2$, $R^3$ and $R^4$ are as defined in claim 1, with the proviso that $-NR^3R^4$ is not piperidino, pyrrolidino, N,N-diethylamino, benzylamino, cyclohexylamino or morpholino when $R^1$ and $R^2$ are hydrogen.

**11.** A compound of formula I according to claim 10, in which $R^2$ is hydrogen.

**12.** A compound of formula I according to claim 10, in which $R^1$ and $R^2$ are hydrogen and $R^3$ and $R^4$ together contain more than 10 C atoms.

**13.** A compound of formula I according to claim 12, in which $R^3$ and $R^4$ are each independently of the other $C_6$-$C_{12}$alkyl or $C_5$-$C_6$cycloalkyl.

**14.** A compound of formula I according to claim 13, in which $R^3$ and $R^4$ are 2-ethylhexyl.

**15.** A compound of formula I according to claim 10, in which $R^1$ and $R^2$ are hydrogen, $R^4$ is a group of formula III and $R^5$ is not arylene.

**16.** A compound of formula I according to claim 15, in which $R^3$ is hydrogen, $C_1$-$C_{24}$alkyl or $C_3$-$C_{24}$alkenyl and $R^5$ is $C_2$-$C_{12}$alkylene.

**Revendications**

**1.** Composition lubrifiante contenant :

a) un ou plusieurs lubrifiants à base d'huiles minérales ou synthétiques,

b) de 0,05 à 5% en poids d'au moins un composé répondant à la formule I :

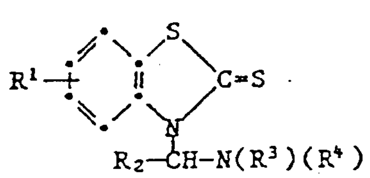

I

dans laquelle
$R^1$ représente l'hydrogène, un alkyle en $C_1$-$C_{12}$, un alcoxy $C_2$-$C_4$, un alcoxycarbonyle à alcoxy en $C_1$-$C_{24}$ ou un nitro,
$R^2$ représente l'hydrogène, un alkyle en $C_1$-$C_{12}$, un furyl-2, un phényle non substitué ou un phényle porteur d'un alkyle en $C_1$-$C_4$, d'un alcoxy en $C_1$-$C_4$, d'un alcoxycarbonyle à alcoxy en $C_1$-$C_{24}$ ou d'un nitro,
$R^3$ et $R^4$ représentent chacun, indépendamment l'un de l'autre, H, un alkyle en $C_1$-$C_{20}$ qui est éventuellement interrompu par un ou plusieurs atomes O, S ou N ou qui porte éventuellement des radicaux oxo ou thiono, un alcényle en $C_3$-$C_{24}$, un alcoxyalkyle en $C_3$-$C_{12}$, un cycloalkyle en $C_5$-$C_8$, un phényle non substitué ou porteur d'un alkyle en $C_1$-$C_{12}$, d'un alcoxy en $C_1$-$C_4$, d'un alcoxycarbonyle à alcoxy en $C_1$-$C_{24}$ ou d'un nitro, un naphtyle, un phénylalkyle en $C_7$-$C_9$, un furyl-2 méthyle ou un (tétra-hydrofuryl-2)-méthyle, ou $R^3$ et $R^4$ forment ensemble, et avec l'atome d'azote auquel ils sont liés, un cycle à 5 ou 6 maillons qui, en plus de l'atome d'azote, peut contenir d'autres hétéroatomes (O, N, S) ou des radicaux oxo ou thiono ou qui peut être condensé à un radical benzo, ou $R_4$ représente un radical répondant à l'une des formules :

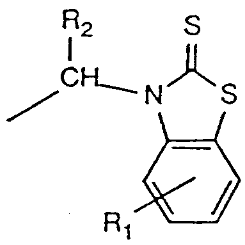 (II) et 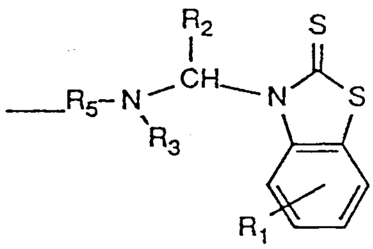 (III)

et
$R^5$ représente un alkylène en $C_2$-$C_{12}$ qui peut être interrompu par O, S ou N ou contenir des radicaux oxo ou thiono, un cycloalkylène en $C_6$-$C_{15}$, un arylène en $C_6$-$C_{15}$, un carbonyle ou un carbothioyle, ou -N($R^3$)-$R^5$-N($R^3$)- représente un radical pipérazinediyle-1,4 qui peut porter un ou plusieurs radicaux méthyles,
comme additifs pour a) diminuant le frottement, l'usure, la corrosion et l'oxydation, et
c) éventuellement d'autres additifs pour lubrifiants.

**2.** Composition lubrifiante selon la revendication 1 qui contient de 0,1 à 3% en poids d'au moins un composé de formule I.

**3.** Composition lubrifiante selon la revendication 1 qui contient un composé de formule I dans lequel la somme des atomes de carbone contenus dans $R^1$, $R^2$, $R^3$, $R^4$ et $R^5$ est supérieure à 10.

**4.** Composition selon la revendication 1 qui contient un composé de formule I dans lequel $R^2$ représente l'hydrogène.

**5.** Composition lubrifiante selon la revendication 4 dans laquelle $R^1$ représente l'hydrogène.

**6.** Composition lubrifiante selon la revendication 3 dans laquelle $R^3$ et $R^4$ représentent chacun, indépendamment l'un de l'autre, H, un alkyle en $C_1$-$C_{24}$, un alcényle en $C_3$-$C_{24}$, un cyclohexyle, un phényle, un phényle porteur d'un alkyle en $C_1$-$C_{12}$ ou d'un alcoxycarbonyle à alcoxy en $C_1$-$C_{24}$, un phénylalkyle en $C_7$-$C_{10}$, un méthoxy-2 éthyle ou un méthoxy-3 propyle, et $R^5$ représente un alkylène en $C_2$-$C_{12}$.

**7.** Composition lubrifiante selon la revendication 1 qui contient un composé de formule :

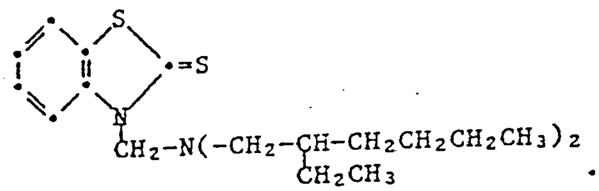

**8.** Composition lubrifiante selon la revendication 1 qui contient un composé de formule :

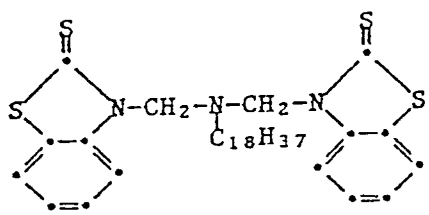

**9.** Composition lubrifiante selon la revendication 1 qui contient un composé de formule :

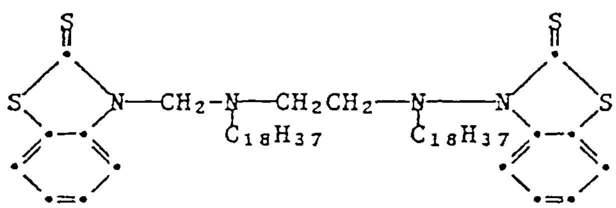

**10.** Composé répondant à la formule I :

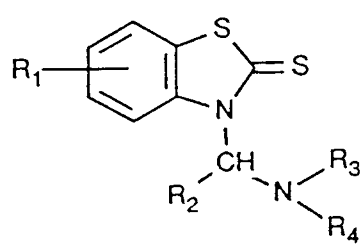

(I) ,

dans laquelle $R_1$, $R_2$, $R_3$ et $R_4$ ont les significations qui leur ont été données à la revendication 1, avec la condition que $-NR_3R_4$ ne représente pas un radical pipéridino, pyrrolidino, diéthylamino, benzylamino, cyclohexylamino ou morpholino lorsque $R_1$ et $R_2$ représentent chacun l'hydrogène.

**11.** Composé de formule I selon la revendication 10 dans lequel $R_2$ représente l'hydrogène.

**12.** Composé de formule I selon la revendication 10 dans lequel $R_1$ et $R_2$ représentent chacun l'hydrogène, et $R_3$ et $R_4$ contiennent ensemble plus de 10 atomes de carbone.

**13.** Composé de formule I selon la revendication 12 dans lequel $R_3$ et $R_4$ représentent chacun, indépendamment l'un de l'autre, un alkyle en $C_6$-$C_{12}$ ou un cycloalkyle en $C_5$ ou $C_6$.

**14.** Composé de formule I selon la revendication 13 dans lequel $R_3$ et $R_4$ représentent chacun un radical

éthyl-2 hexyle.

**15.** Composé de formule I selon la revendication 10 dans lequel $R_1$ et $R_2$ représentent chacun l'hydrogène, $R_4$ représente un radical de formule III et $R_5$ un radical arylène.

**16.** Composé de formule I selon la revendication 15 dans lequel $R_3$ représente l'hydrogène, un alkyle en $C_1$-$C_{24}$ ou un alcényle en $C_3$-$C_{24}$ et $R_5$ représente un alkylène en $C_2$-$C_{12}$.